(19) Europäisches Patentamt
European Patent Office
Office européen des brevets

(11) Publication number: **0 009 222**
**A2**

# (12) EUROPEAN PATENT APPLICATION

(21) Application number: 79103451.5

(22) Date of filing: 17.09.79

(51) Int. Cl.[3]: **C 12 Q 1/00**, C 12 Q 1/32, C 12 Q 1/50, C 12 N 9/96, C 12 Q 1/52, C 12 Q 1/54, C 12 Q 1/58

(30) Priority: 20.09.78 US 944192

(43) Date of publication of application: 02.04.80 Bulletin 80/7

(84) Designated Contracting States: BE CH DE FR GB IT LU NL SE

(71) Applicant: American Monitor Corp., P.O.Box 68505, Indianapolis Indiana 46218 (US)

(72) Inventor: Denney, Jerry W., 45 Thornhurst Drive, Carmel, Indiana (US)

(74) Representative: Speidel, Eberhardt, Postfach 1320, D-8035 Gauting (DE)

(54) **The stabilization of working reagent solutions containing NADH, NADPH, and/or enzymes, and the use of such stabilized reagents in enzyme or substrate assays.**

(57) Prolonged stabilization (at least one month) of working reagent solutions of NADH (or NADPH) and of enzyme solutions, including both those used in measurement of enzymes and those in which enzymes are included for measurement of a non-enzyme substance, and stabilization of combinations thereof in certain assay systems.

EP 0 009 222 A2

0009222



# THE STABILIZATION OF WORKING REAGENT SOLUTIONS CONTAINING NADH, NADPH, AND/OR ENZYMES, AND THE USE OF SUCH STABILIZED REAGENTS IN ENZYME OR SUBSTRATE ASSAYS

## I. Background of the Invention: Enzymes, Their Use and Measurement:

### I.A. Introductory Comments of Background, and of Vital Significance of Enzymes:

Enzymes have been used for many years, in reagents in various biological and chemical assays; and these many uses of enzymes have long included the quantitative measurement of a wide variety of substances in blood, tissue fluids, and in other substances in which measurements of constituents are desired.

The measurements involving enzyme activity have thus been long in use for vital diagnostic medical purposes and for medical research; for enzyme reactions have been long known to be reactions by which the measurement or quantitation of diagnostically-significant body substances can be done.

These assays which involve enzyme reactions are, accordingly, of very useful and medically-vital purposes; for they are useful in the quantitative measurement of blood enzymes and enzymes of other body fluids, and also to give a quantitative measurement of even non-enzyme substances.

However, a particular and long-standing problem of the attempts at accurate measurement of many blood enzymes is that unfortunately they manifest no easily-perceived indication of their presence or activity; and thus in these instances their activity is coupled with reagent enzymes which are of a type or nature which do have an associated measurable change incident to their reaction.

I.B. Enzyme Systems of Coupled Nature Illustrate Enzyme Procedures:

The "coupling" of one enzyme to another is an indirect yet very useful reaction, used in assay techniques; and it involves the allowing or use of a product of a first enzyme reaction to serve as a substrate or intermediate product upon which a second enzyme acts, producing yet another product, and one of the products of the second reaction is the thing whose measurement provides in effect a desired quantitation or measurement of the particular factor of interest of the first reaction.

As an illustrative example, the directly-measurable factor of a coupled enzyme reaction is a change in the spectrophotometric absorbance due to the production or consumption of the substance NADH (nicotinamide adenine dinucleotide, reduced form) or NADPH (nicotinamide adenine dinucleotide phosphate, reduced form) which is quantitatively measurable by its characteristic of absorbing strongly in the ultraviolet region of the spectrum, and thus the measured change of that absorbance is useful to give the quantitative indication of the NADH reaction involved.

I.B.1 Coupled Enzyme System to Measure Enzyme SGOT:

An example of the indirect measurement of an enzyme, of a coupling enzyme-reaction type, is the indirect yet quantitative measurement of the very significant blood enzyme SGOT (serum glutamic-oxaloacetic transaminase). That enzyme SGOT is a substance which is very important medically,

for it is released into the blood stream when a person's heart is damaged; and therefore it has the vital medical-diagnosis significance of being a measure of the incidence and/or extent of myocardial infarction (commonly called "heart attack").

And, as has been long known, a prompt and accurate measurement of apparent heart damage is vital as a means of diagnosis and treatment of the heart malfunction; and since treatments vary according to what is the nature and extent of the specific malfunction which causes the heart-damage symptoms, the prompt and accurate SGOT measurement is of vital health-improving and even life-saving significance to the patient.

The enzyme SGOT is measured by its role in catalyzing the first of the reactions mentioned below, and a second enzyme MDH (malic dehydrogenase) is used to catalyze a reaction involving a product of the SGOT reaction; and thus these two reactions illustrate not only a vitally-significant measurement of SGOT and apparent heart damage, but illustrate also the use of one enzyme (MDH) to measure another enzyme (SGOT) in an indirect or coupled enzyme system reaction phenomenon. The SGOT reaction may be represented as follows:

aspartic acid + alpha ketoglutaric acid $\xrightarrow[\text{pyridoxal-5'-phosphate}]{\text{SGOT}}$ glutamic acid + oxaloacetic acid

However, an indirect method of measurement is here required, because none of the participating substances for this SGOT reaction nor the products of that reaction are easily measured. Thus the measurement of the diagnostically important SGOT activity must be coupled with a second reaction which is easily measured; and commonly that second reaction is one using the enzyme MDH as a catalyst, as follows:

$$\text{oxaloacetic acid} + \text{NADH} \xrightarrow{\text{MDH}} \text{malic acid} + \text{NAD}$$

The loss in ultraviolet absorbance of NADH, incident to its conversion to NAD, is something which is easily measured; and thus this reaction, catalyzed by the MDH, provides the desired although indirect quantitation of the activity of the amount of SGOT in the blood, and thus gives the observer the vital data as to the heart damage.

I.B.2 <u>Coupled Enzyme System Using LDH to Measure Enzyme SGPT:</u>

Another illustration of a coupled enzyme system, using one enzyme (LDH) to measure another enzyme (SGPT), is that of the indirect assay for SGPT, vitally significant in diagnosis of liver diseases as well as diagnosis of myocardial infarction, as follows:

$$\text{alpha ketoglutaric acid} + \text{L-alanine} \xrightarrow{\text{SGPT}} \text{L-glutamate} + \text{pyruvate}$$

Similar to the SGOT example given above, none of the participating substances for this SGPT reaction nor its reaction products are easily measured, and thus a second reaction using LDH as catalyst must be coupled with the first reaction to get an easily measurable product, as follows:

$$\text{pyruvate} + \text{NADH} + H^{+} \xrightarrow{\text{LDH}} \text{Lactate} + \text{NAD}^{+}$$

The loss in ultraviolet absorbance of NADH is easily measured; and this reaction, catalyzed by LDH, provides a useful although indirect quantitation of the amount of SGPT in the blood.

I.C. Enzymes in Measurement of Non-Enzyme Substances:
Enzymes are also used as reagents to measure non-enzyme substances which are not easily detected by ordinary color-producing dye reactions and/or in cases where other substances are mistakenly measured by such reactions as supposedly being the substance for which the measurement was intended. More particularly, the known high specificity of enzymes is used to convert substances to be measured in the blood to a compound which can be easily and specifically measured.

In other cases, the enzyme reagent produces an easily measurable change while converting the substance of interest in whatever is the particular reaction. The widely used hexokinase measurement of blood glucose is an example:

$$\text{glucose + ATP} \xrightarrow{\text{hexokinase}} \text{glucose -6-phosphate}$$

$$\text{glucose-6-phosphate + NAD} \xrightarrow{\text{G-6-PDH}} \text{NADH+ gluconolactone-6-phosphate}$$

Thus, two enzyme reagents are used to convert glucose to gluconolactone-6-phosphate. The NADH formed absorbs ultraviolet light strongly; and the spectrophotometric absorbance at 340 nm is thus useful as a measurable determination of the glucose concentration in the blood sample used.

II. The Vital Significance of Long Stability of Enzymes as Reagents:

II.A. Magnitude:
A wide variety of diagnostic tests or assays have for many years been performed in hospital and clinical laboratories, using enzymes as reagents. About 300 million such diagnostic tests are performed on blood samples by such laboratories each year in the United States; and the quantity

approaches a billion of such tests annually worldwide.

II.B. Stability Distinctions between Dry Stock Enzyme Reagents and that of Working Reagent Solutions:

Because of the stability problem (short "shelf life") of enzymes in the liquid solution form in which they are used, reagent enzymes used in these tests are generally supplied in the dry form. This "dry form" storage method, although beneficial in prolonging the enzyme stability, has a great disadvantage of causing the laboratory to have to then reconstitute the enzyme reagent with water to obtain a working reagent for use in the assay. (A "working reagent", more fully described below, is one which is added to the diagnostic test directly, without additional preparation.) Working enzyme reagents are desirably used in these tests or assays in the form of a liquid solution, for reasons such as ease of handling and measured delivery; but in such form of liquid solution such enzyme reagents are characteristically stable for only a few hours and at most a few days, even under refrigeration. And use of enzyme reagents at the more convenient room temperature may drastically reduce stability even further.

II.C. " Stability " Herein Used in Month-long Sense:

Stability of working enzyme reagents is herein meant to refer to the retention of the utility of those enzyme reagents in performing clinical assays at some period of time (generally being about one month after preparation) in yielding clinically-useful diagnostic results.

II.D. Known Instability Disadvantages of Working Reagent Solutions Here Overcome:

At best, the instability of relatively expensive enzyme reagents creates economic waste, since the unused reagent must be discarded. At worst, the unstable reagent may cause a mistaken diagnostic measurement to be made; for

enzyme reagents which have lost activity may cause falsely elevated or decreased measurements. Deterioration may also have the effect of altering the range of the assay, so that a severe abnormality may be reported as normal or only slightly elevated; and this of course causes a wrong diagnosis which may have many dangerous or at least bothersome effects.

Further, the short stability of course leads to the relatively expensive and bothersome laboratory procedure of reagent-preparation in relatively small batches.

These many disadvantages of short-stability of enzyme reagents seem so evident that further emphasis seems unnecessary, except perhaps to mention that false assay valuation may of course result in missing the detection of a disease state in a patient or in falsely diagnosing a disease state which does not exist. And although early diagnosis of disease is of course generally believed to be desirable, erroneous diagnostic information may delay proper diagnosis, and may cause expensive, damaging or uncomfortable treatment to be given for a disease which does not exist.

Such disadvantages of short-stability enzyme reagents have been long realized.

In spite of all this, increasing dependence upon laboratory diagnostic blood tests in the practice of medicine has caused the prior art both to use short-stability enzyme reagents in spite of these disadvantages, and to attempt to utilize various types of procedures and concepts to lengthen enzyme-stability to as long as possible, even though such attempts have generally not accomplished working reagent stability for more than a few hours or at most a day, even under refrigeration.

III. Prior Art as to Stabilization of NADH Reagents; and Comments as to NADH Instability:

Reagents containing NADH pose two particular stability problems which are advantageously overcome by the concepts of this invention.

As to the problems of NADH stability, the prior art has suggested that NADH solutions are more stable at akaline pH levels as contrasted to acid pH values. Also it is known that NADH is more stable in Tris buffer used in working reagents than in water. (The Tris buffer is believed to be commonly used in a concentration of about 0.1 M for purpose of maintaining pH of the enzyme assay.) However, the prior art has not produced an NADH working reagent solution which is stable and free from one or both types of deterioration, described below, for prolonged periods of time.

As mentioned above, the property which makes NADH (and the NADPH) useful in coupled enzyme reactions is its absorbance of ultraviolet light. A first source of instability in NADH is the loss of optical density (O.D.) in a solution of NADH, due to loss of ability to absorb ultraviolet light, the O.D. of a solution being a function of its ability to absorb ultraviolet light. The result is a loss of ability to detect enzyme activity.

For example, a freshly prepared solution may have a O.D. of 2.0. Such a solution might detect enzyme or substrate levels which produced a change from 2.0 down to 0.5 O.D. If, because of aging, the O.D. of the working reagent solution decays to 1.0 O.D. from 2.0, the reagent system can only measure one-third (that is, 0.5 O.D./1.5 O.D.) as high a level of enzyme or substrate.

If a fresh reagent system for SGOT were capable of detecting abnormal levels of three times normal, such decay

would allow measuring only one times normal, and abnormal levels would not be detected; and since levels of three times normal would be seen in a severe heart attack, whereas normal SGOT levels are rarely seen in severe heart attacks, the physician might be led to a tragically mistaken diagnosis.

A second type of deterioration in working reagents containing NADH is the formation of inhibitors. (The loss of O.D. type of deterioation is believed by applicant to happen either with or without inhibitor formation; and inhibitor formation is believed by applicant to occur either with or without loss of ultraviolet (U.V.) absorbance.)

Inhibitor formation in NADH solution causes a lowering of rate in the enzyme reactions which which the NADH is coupled. As an Example, a NADH working reagent solution which has deteriorated by way of inhibitor formation, if used with even fresh working reagents containing the other ingredients for an SGOT test, will produce lower apparent SGOT results. Consequently, a laboratory might mistakenly report a normal SGOT value on a patient with acute heart disease even though he has an elevated blood level of SGOT.

IV. <u>Characteristics or Nature of Enzyme Instability; and Prior Art as to Enzyme Stabilization Attempts:</u>

IV.A. <u>Instability of Working Solutions in Prior Art:</u>

It is known, as mentioned above, that when enzymes are in aqueous solutions, they rapidly lose their activity. Thus, reagents so prepared "decay" because of loss of enzyme activity, and after a short period of time, they cannot and should not be used, due to such activity loss; for a loss of enzyme activity, of such significant amount, often occurs in as short a period of time as a few hours, even when the enzyme reagent is refrigerated.

Refrigeration of aqueous solutions of enzymes has been known

by the prior art to relatively prolong stability to a certain extent. However, even under careful refrigeration, enzyme solutions generally lose substantial activity in only a few hours or at most one day.

IV.B. Inherent Nature of Enzyme Instability:

Indeed, since all enzymes come from natural sources such as plant, animal, and microbial life, it seems that the well-known property of instability of enzymes in aqueous solutions is inherently natural and vitally important in such life forms, even though the present inventive concepts achieve a result quite opposite to this apparently-natural characteristic of enzymes.

In nature, enzymes are used by living organisms to synthesize and catabolize virtually every compound making up the organism, as well as to control energy utilizations; and an important means which seemingly is inherently employed in nature to control such life-processes is the control of enzyme production or synthesis. If, in nature, enzymes were totally stable in the aqueous media which makes up the internal environment of all life, their level would never be reduced, only increased, and consequently control of metabolic processes would be lost.

IV.C. Working Reagent Solutions are the Desired Form for Use:

As a result of this inherent and natural enzyme instability in an aqueous solution, considerable amount of time must be spent in preparation of fresh aqueous enzyme reagent systems known as "working reagents". A working reagent is one which is used in an "as is" condition in an enzyme assay test without additional preparative steps such as adding dry, salt-suspended, or glycerol-stabilized enzymes. A further characteristic of a "working reagent" is that the ingredients of the reagent are in aqueous solution, as contrasted to being in suspension or glycol/water solution.

In addition, working reagents should be of such volume as to be conveniently and accurately added to the enzyme assay mixture in the course of performing an assay. In particular, very small volume additions are cumbersome, time-consuming, and not as likely to be accurate as are larger volumes, either in manual or automated assay methods.

To minimize volume measurement error, it is desirable that each reagent addition be as large a proportion of the final volume as possible. That is, if two reagents are used, it is generally desirable that each be about one-half of the total final volume.

In addition to "working reagents" being desirable from the standpoint of efficiency due to elimination of added steps and minimization of volume error, working reagents are conducive to repeatability of ingredients from one assay to another, which repeatability may be vital to accurate assay from one patient to another.

The enhanced reproducibility of working reagents is due to the fact that ingredients are in solution in working reagents, rather than being dry or in suspension. Solutions by definition are uniform in concentration from one portion of the liquid to another.

By contrast, suspensions are not homogenous; and consequently, particularly if a small sample is used in each test, the sample may differ in the amount of suspended ingredient. (An extreme example of this phenomonon is represented by taking samples of water containing suspended sand. Each sample of exactly equal volume does not necessarily contain exactly the same number of grains of sand.)

The raw material enzymes used by the prior art to prepare working reagents have been stabilized by drying, in

glycerol solutions, and by suspension in concentrated (3-6 equivalents per liter) of ammonium or sodium as sulfate salts. Although useful in stabilizing raw materials, these means are not usable in stabilizing working reagents, and the working reagents produced such raw materials by the prior art are unstable. Dry enzymes are not easily dispensablė and must got into solution prior to use, and cannot easily be used as working reagents. Enzymes in glycerol are difficult to use as a working reagent because glycerol is very viscous; and dispensing, mixing, and bubble inclusion are problems in spectrophotometric observations. Enzyme suspensions cannot be used as working reagents because the enzymes contained are not in solution, and accurate sampling is a problem. Further, the high concentrations of ammonium or sodium sulfate would likely adversely affect the properties of the enzyme assay for which the working reagent is intended.

IV.D. Enzyme Instability Summary:
Thus, instability seems to be natural and a fundamental property af enzymes. Although the instability property seems desirable *in vivo* in nature, the inherent instability of enzymes is indicated above as being generally undesirable in an *in vitro* use of enzymes in assay methods.

IV.E. Enzyme Instability Generally Accepted by Prior Art as to Working Reagent Solutions:
Indeed, it seems even that the well-known natural and inherent instability of enzymes in aqueous solutions has been taken for granted by the prior art when employing enzymes as reagents. And refrigeration (which slows most chemical and biological processes) has been used to slow the process of decay somewhat, although to only a few hours.

The prior art acceptance (or perhaps its widespread recognition, acquiescence, and even resignation of or to the

fact) of the unstable nature of enzymes has apparently led to a corresponding and apparently full-scale acceptance by the prior art of the use of working enzyme reagents with only a few hours stability.

IV.F. Mitchell is Significant Exception to Prior Art's Acceptance of Instability, Even Though Quite Different from the Present Invention:

A significant prior art exception has been the attempt of Mitchell (U.S. Patent No. 3,962,037) to enhance the stability of enzyme-containing reagents for the measurement of glycerol. Mitchell used dextran and Cleland's reagent to achieve satisfactory storage periods of a few days in the glycerol measuring system, relative to the goals of that invention, when the reagent is refrigerated.

The means taught by Mitchell differs from the present invention, in addition to other ways, in that it accomplishes only a few day's stability; and it further appears limited to use in a particular assay system (i.e., glycerol).

Also, in the Mitchell patent, the use of a strong reducing agent (Cleland's reagent) apparently eliminates application where NADH is not measured by U.V. absorption but is coupled to a redox dye such as INT, 2-(p-Iodophenyl)-3-(p-nitrophenyl)-5-phenyl Tetrazolium or redox indicating copper or iron legends. In contrast, the present invention does permit that special type of NADH quantitation, if desired.

Additionally, Cleland's reagent *per se* is a probable contributor to instability of some enzymes.

V. The Present Invention Concepts (as to the Stabilization of NADH and NADPH); and Further Contrasts to Prior Art:

V.A. Inventive Concepts as to Inclusion of Base, Amount

and pH as Factors:

The present invention, with respect to the advantageous stabilization of working reagent solutions of NADH and NADPH, involves the addition of bases to aqueous solutions of NADH or NADPH, according to two significant discoveries as to the addition of the bases. That is, the amount of base required is discovered to be critical in amount. Further, the pH of the NADH or NADPH solution is discovered to be also critical.

V.B. Stability Achievement for NADH and NADPH in Working Solutions:

These two concepts, and particularly in combination (that is the critical concentration of base and the critical pH of the NADH or NADPH solutions) accomplish the heretofore-unachieved but highly advantageous goal of providing stability of the NADH or NADPH solution for one month.

That is, the lack of stability of NADH or NADPH by virtue of loss of ability to absorb ultraviolet light (measured as loss of absorbance at 340 nm), and also their lack of stability by virtue of inhibitor formation, are successfully eliminated by the present invention.

As used herein, a reference to a NADH or NADPH solution refers to a solution of NADH or NADPH, respectively, in water, which may be prepared by dissolving either NADH or NADPH, as the case may be, in water, or making a NADH or NADPH salt solution in water. (It seems that the commercial supply of NADH or NADPH is usually in one or more salts thereof.)

V.C. Fortuitous Use of NADH and NADPH in Prior Art Buffers:

It is acknowledged that NADH and NADPH were included, although fortuitously, by the prior art in the buffer required to regulate the pH of the enzyme which was to be

assayed. Thus to the extent that the prior art fortuitously employed amine bases at alkaline pH's, in the preparation of buffers, and included NADH or NADPH in these buffers, the prior art had included NADH or NADPH with alkaline amine. And since it was recognized by the prior art that such inclusion of the NADH or NADPH in the buffer resulted in some degree of stability-enhancement (although not the kind of enhancement of stability achieved by the present invention), one might assume that the prior art would assume that more of the buffer (or perhaps by even a further extension of such logic that more of one of the ingredients of the buffer), would stabilize even more.

V.D. Details Showing that the Present Concepts Distinguish from the Prior Art's Fortuitous Use in Buffers: However, quite to the contrary, it was discovered as a part of the concepts of the present invention that only critically small amounts of base are optimal to achieve the stability and operability goals of this invention, quite in contrast to the prior art.

In further consindering the present inventive concepts, and by way of further contrasting the present discovery with the prior art, it may be helpful to note that the present invention distinguishes from the prior art in both the quantitative and qualitative aspects of the prior art's adventitious inclusion of NADH or NADPH in buffer systems. As to the qualitative aspects of such prior art's inclusion, these were seemingly unrecognized by the prior art; and as to the quantitative aspect, this was undiscovered by the prior art, and the inter-relationships between the quantitative and qualitative aspects were also undiscovered by the prior art. And compounds were included by the prior art, as a part of such buffers, which were not only of no benefit as to stability but probably even detracted from

the stability of the resulting NADH-NADPH solutions of the prior art.

V.E. <u>The Nature of Buffers Helps Discredit the Fortuitous Prior Art as to Inclusions of NADH or NADPH in Buffer:</u> The significance of this undesirable inclusion qualitatively by the prior art, particularly its inclusion of other substances (other than amine base) as a part of the prior art's inclusion of NADH or NADPH in a buffer solution, may be perhaps most easily seen by considering briefly just what is a buffer. This also may help in realizing the significance of the present invention's non-inclusion of substances other than base.

A buffer is a solution of a weak acid and its conjugate base, or a weak base and its conjugate acid. Amine bases are weak bases, and buffers are made from them by adding an acid such as hydrochloric acid so that a substantial amount of the amine base forms a new compound, the corresponding conjugate acid of that amine base. The resulting solution contains substantial amounts of both amine base and the conjugate acid of the amine base.

The amount of conjugate acid of the amine base relative to the amount of amine base *per se* may be approximated by a mathematical expression related to the Henderson-Hasselbalch equation:

$$pH=pK_w - pK_b + \log \frac{[B]}{[HB]}$$

where:

$K_w$ is the dissociation constant of water

$pK_b$ is the dissociation constant of the amine base

[B] is the concentration of amine base

[HB] is the concentration of the conjugate acid of the amine base

TRIS is an amine base widely used to prepare buffers. Buffers used in enzyme assays are commonly employed at pH 7.4 at a concentration of at least 0.1 M. The $pK_b$ of TRIS is 5.76. In a prior art use of NADH in such a buffer, the relative concentration of conjugate acid of the TRIS to the base form of the TRIS may be calculated by the above equation as follows:

$$7.4 = 14 - 5.76 + \log \frac{[B]}{[HB]}$$

$$\log \frac{[B]}{[HB]} = -0.84$$

$$\frac{[B]}{[HB]} = 0.144$$

or

$$\frac{[HB]}{[B]} = 6.9$$

Thus, in such a buffer, the concentration of the newly formed of the TRIS relative to the concentration of the TRIS base is about 7 times as great. Thus the buffer contains about o.087 moles per liter of newly formed conjugate acid of the TRIS and 0.013 moles per liter of the TRIS base. Since a strong acid was used by the prior art to adjust the pH of the TRIS, the concentration of anion of the strong acid (usually chloride) would be about 0.087 M, since the hydrogen ion of the conjugate acid of the TRIS comes from the strong acid.

V.F. Specific Disadvantages of Prior Art's Fortuitous Use of NADH or NADPH in Buffer:

The prior art inclusion of NADH in a buffer solution thus included large amounts of conjugate acid of the amine base (usually TRIS) from which the buffer was prepared as well as large amounts of anion (usually sulfate or chloride) of the acid used to adjust the pH of the buffer. Also, high concentrations of the amine base were present.

According to the optimal application of the present inventive concepts, only the NADH or NADPH and base are present, and only a trace of conjugate acid of the base is present as a consequence of acid contributed by the NADH or NADPH itself. Also no other anions need be present, and desirably are even absent, in contrast to those anions inherently present in the buffer solution of the prior art.

Further, in addition to the prior art's using too much of an amine base in a buffer system, the prior art used the amine base in a buffer system at an undesirable pH for stability enhancement; that is, the prior art used the amine base in a buffer system at the pH at which the enzyme assay was performed, which pH levels were generally not optimum according to the present invention for stabilizing the NADH or NADPH.

Although it apparently was pragmatic and apparently obvious for the prior art to use the buffer at the pH of the assay (even though now discovered to be not desirable from a stability standpoint), the present inventive concepts significantly depart from the prior art in departing from the pH of the assay as being a criterion for the pH of the working reagent solution of NADH or NADPH stabilized by the concepts of the present invention.

Indeed, should one attempt to apply the concentrations of

amine base buffer used by the prior art, to one of the inventive concepts of the present invention, (i.e., the departure from using the pH of the assay as a criterion) not only would the NADH or NADPH solution have less stability than according to the optimal practice of the present invention, but the high concentration of buffer at pH' employed in the present invention would likely adversely affect the pH at which the enzyme assay is conducted when the NADH or NADPH working solution is mixed with other working reagents.

V.G. <u>Inventive Concepts as to Stabilization of NADH or NADPH Summarized:</u>

Thus the combination of critically small amounts of base and optimal pH of the NADH or NADPH working reagents not only accomplishes stability of that working reagent, but also allows the combination of that reagent with other working reagents of that assay in a workable fashion.

V.H. <u>Concepts as to Bases Used in Stabilization of NADH and/or NADPH:</u>

Further, with reference to such bases, the present invention uses amine bases including triethanolamine, diethanolamine (DEA), ethanolamine, tris (hydroxymethyl) aminomethane, imidazole, 2-amino-2-methyl-1-propanol, 2-amino-2-methyl-1,3-propanediol, and strong bases including ammonium hydroxide, sodium hydroxide, potassium hydroxide, lithium hydroxide, magnesium hydroxide, and calcium hydroxide. The present invention also uses basic solutions of salts that are formed from the combination of a strong base and weak acid such as rubidium carbonate, sodium borate, sodium carbonate, calcium carbonate, and ammonium carbonate. Although effective in stabilization, calcium and magnesium bases and salts are less desirable because of their limited solubility at the pH discovered to be optimal according to the concepts of the present

invention.

V.I. Relative Ranking of Bases:

It was discovered as a part of the present invention that cartain bases are generally more effective than others. From most effective to least effective, they are: ammonium hydroxide, triethanolamine, tris (hydroxymethyl) aminomethane, magnesium hydroxide, calcium carbonate, ammonium carbonate, sodium borate, sodium carbonate, rubidium carbonate, lithium hydroxide, sodium hydroxide, potassium hydroxide, imidazole, diethanolamine, 2-amino-2-methyl-1-propanol, 2-amino-2-methyl-1,3-propanediol, and ethanolamine.

V.J. Optimal Stabilization:

According to the present invention, it was discovered that it is only when such critically small amounts of bases are combined with NADH or NADPH within a limited pH range, desirably in the absence of significant amounts of the conjugate acid of the amine base or other ions included incidental to the preparation of the conjugate acid of the amine base, that optimal stabilization is achieved.

In particular, the present invention uses the minimum amount of base required to adjust the pH of a NADH or a NADPH solution to a pH of at least about pH 7.9.

V.K. Considerations in Selection of pH:

Practical considerations will easily lead the practitioner of the present invention to choose the most suitable pH above the minimum pH of about 7.9 in a particular application of the invention to a working NADH or NADPH solution. As an example, the use of a pH of 10 in conjunction with the other inventive concepts of the present invention may produce a working reagent which requires very strong buffering in other working reagents with which it is to be

combined, and thus may be less desirable even though it is effective in stabilizing the NADH working reagents solution.

The pH values of 8.3 - 8.5 have been found to be practical and widely applicable in a number of specific embodiments of the present invention, where the stable NADH or NADPH working reagent is used with a wide variety of working enzyme reagents.

VI. The Present Inventive Concepts (As to the Stabilization of Enzymes in Working Reagents); and Further Contrasts to the Prior Art:

VI.A. The Invention as to Salt-Stabilization of Enzymes: The present invention employs the proper concentrations of a variety of organic and inorganic salts to stabilize important enzymes useful in the measurement of medically important enzymes and other substances in the blood and other body fluids.

Further, with reference to such salts, the present invention uses sodium, potassium, rubidium, ammonium, lithium, or basic amine cations, in combination with organic monophosphate esters (including glycerol monosphate, ethylene glycol monosphate, and creatine phosphate), sulfate, certain sulfonated organic acids, carbonate, phosphate, borate, certain dicarboxylic acids, certain dicarboxylic amino acids, or certain monocarboxylic acids, as anions. The concentration of the salt used is generally optimal at a cation concentration of about one equivalent per liter.

It was discovered as a part of the present invention that certain cations, among the several workable cations, are more generally effective than others. From most effective to least effective, they are rubidium, potassium, sodium, ammonium, amine bases, (i.e., TRIS and TEA and imidazole), and lithium ions. Among the anions, sulfate, phosphate

esters including alpha or beta glycerophosphate, ethylene glycol monosphate, dicarboxylic acids (i.e., malate and succinate), sulfonated organic acids such as PIPES and phospate are best at stabilizing, followed by carbonate and borate, and dicarboxylic amino acids including alanine and aspartic acid, while monocarboxylic acids (i.e., acetate) are least effective.

The differences between all of the anions above are not as great as the differences in effectiveness of cations, except that monocarboxylic acids are far less effective than the other anions.

Halogen anions such as chloride and bromide are not workable in the present invention. The diphosphates of ethylene glycol and glycerol are not effective.

In general, the invention is widely applicable to the stabilization of enzymes. The particular cation and anion used and the concentration of the salt formed from particular cation and anion from those found to be effective within this invention, are determined by practical considerations, such as how they affect the rate of the enzyme stabilized or the enzyme being measured.

As an example of choosing salt concentration, hexokinase and glucose 6-phosphate dehydrogenase (G-6-PDH) are stabilized by 0.45 M potassium sulfate; however, the rate of reaction with ATP of these two enzymes is markedly diminished. Thus when using these enzymes to measure ATP in a working reagent, or ATP produced by another enzyme in a working reagent (such as in CPK assay) the concentration of the salt must be diminished as a compromise between the stability of the working reagent and the rate of ATP measurement.

It will be understood, in practicing the invention to stabilize a particular enzyme system, that one should choose a combination of anions and cations taught herein to be workable as a part of this invention which provides the best results in providing a stable useful enzyme reagent.

In certain applications of the present invention, where an anion taught to be workable according to the present invention is a substrate in the enzyme assay for which the working enzyme solution is to be stabilized (i.e. aspartate or alanine in the SGOT or SGPT enzyme assay) the proper cation is chosen from those taught to be useful according to the present invention to compliment the carboxylic amino acid anion of the substrate.

Also, combinations of workable anions and cations may be used. A carboxylic acid and sulfate may be used as the anion, and potassium and TRIS may be used as the cation, yielding a mixture of salts known to be useful as a part of the inventive concepts of this invention.

Although carboxylic acids are generally useful, in employing the invention with respect to particular enzyme working reagent systems, it will be apparent to one skilled in the art that a carboxylic acid must have certain properties, such as that it is soluble, does not react with other substrates or ingredients, does not complex metals required for the activity of the enzyme assayed or of the enzyme used as a reagent in an assay in which such complexing would adversely affect the reaction, does not have oxidation or reduction properties incompatible with the reagent system or the enzyme assay, does not have absorbence in the spectral range used in the assay, and does not react with the product of the assay, and is not an inhibitor of enzymes used in the assay or of an enzyme being

assayed.

The same consideration as described for the carboxylic acids apply when choosing a particular amini base or sulfonated organic acid when practicing the invention.

Further, one should not choose a combination of anions and cations which includes one of the products of the enzyme reaction(s).Thus, malic acid would not be chosen to stabilize the enzyme in a working reagent for SGOT using malic dehydrogenase. Ammonia is not useful in stabilizing reagents for urea assay using urease and glutamate dehydrogenase because it is an intermediate in the assay. Obviously, ammonia is not included to stabilize glutamate dehydrogenase used to measure ammonia.

VI.B. <u>Contrasts to Prior Art; Working Reagent Stabilization Rather Than Merely of Stock or Preparatory-state Reagent Supply:</u>

The novel concepts of the present invention achieve the heretofore unachieved goal of stabilizing a variety of enzymes in working reagent solutions.

This contrasts with the prior art which has not achieved prolonged stability in working reagent solutions but only achieved prolonged stability in stock reagents which were then used to prepare working reagents solutions.

The prior art's use of salt is a further contrast to the present invention. Not only did the prior art achieve only stabilization of stock reagents with salts as opposed to the present invention's advantageous stabilization of working reagents, but also there are significant qualitative and quantitative differences in such prior art's use of salts.

VI. C. Contrasts to Prior Art, Qualitative:

The prior art's stabilization of stock solutions used sodium and ammonium sulfate to suspend enzymes which were not generally in solution. By contrast, the present invention teaches the use of a variety of both anions and cations, not merely sodium, and ammonium sulfate, to be useful in the stabilization of enzymes in solution in working reagent. The present invention teaches that in many instances there are better and/or more useful combinations of these anions and cations than ammonium or sodium sulfate. The present invention teaches a general hierarchy among such useful anions and cations which may be combined in various combinations and mixtures. In particular, certain of the cations may be more advantageous in certain applications than sodium or ammonium ions.

VI.D. Contrasts to Prior Art, Quantitative:

Further, the present invention with respect to the stabilization of enzymes in working reagent solutions contrasts quantitatively from the prior art's use of salts to stabilize enzymes in stock suspensions in that the prior art used two to six equivalents per liter of sodium or ammonium cation with a corresponding amount of sulfate anion to stabilize such suspensions. The present invention utilizes cation concentrations of only about one equivalent per liter with corresponding anion concentrations. In several specific embodiments, cation and anion concentrations are far less than one equivalent per liter.

VI.E. Contrasts to Prior Art, Combination with NADH Stabilization:

Further contrasting the present invention from the prior art, the present invention has been shown to be useful in combination with novel means of stabilizing NADH or NADPH in enzymatic assays.

VI.F. Enzyme Stabilization Without Altering Physical Properties

Also, the present invention accomplishes the goal of stabilizing working enzyme reagents without the necessity of significantly altering the physical properties of the working enzyme reagents. That is, the working reagents prepared according to the concepts of the present invention contain no ingredients in suspension as opposed to being in solution, need not show significantly altered viscosity, and may be prepared so as to show insignificant differences from water in refractive index. Such unaltered physical properties have significant advantages when such reagents are used in enzyme assays, particularly when used in automated assays.

VI.G. Stability-Testing Procedures:

Stability testing of the concepts and embodiments set forth herein, relating to stability of working reagent solutions of the NADH (or NADPH) and also of the various enzymes, were made both by the actual storage of working reagent solutions thereof for at least one month under refrigeration at 4°C, and/or by the accelerated method reported by Lordi and Scott (J. of Pharm. Sci., Vol. 54, No. 4, p. 531-537). (Certain of the solutions were stability-tested by both methods, for purposes of control and cross-checking, and the accelerated method of Lordi and Scott was shown to be sufficiently reliable, within experimental limits; and the stability was found in all cases, which were tested by only the accelerated method, to be sufficiently prolonged that any differences, between the results given by the accelerated method and by the actual calendar-stability term, would be of negligable import in consideration of the one-month stability achievement.)

## VII. EMBODIMENTS AND SPECIFIC TEST PROCEDURES FOR STABILIZATION OF WORKING REAGENT SOLUTIONS:

### VII.A. Embodiments of Stabilization of NADH and NADPH:

Several embodiments illustrate the inventive concepts:

#### VII.A.1. NADH Stabilization:

a. Stabilized NADH I:

A stabilized NADH reagent was prepared by dissolving 0.188 grams of beta nicotinamide-adenine dinucleotide, reduced form, disodium salt, dihydrate (NADH) and 25 milligrams of chloramphenicol (an antimicrobial) in approximately 950 ml of deionized water. The pH of this solution was adjusted to 8.3 with approximately 0.2 ml of a solution of 5% TRIS, that is, 5 grams of TRIS in 100 milliliters of deionized water; and the reagent made to final volume of one liter with deionized water, which made a solution of approximaterly 80 micromolar TRIS. This solution was filter sterilized, and was determined to be stable for at least one month when stored at 4°C.

(The filter sterilization, as referred to herein, was by drawing the solutions by suction through a Nalge filter with a pore size of 0.22 microns and into a sterile container for storage.)

b. Stabilized NADH II:

A stabilized NADH solution was prepared by dissolving 0.188 grams of NADH, 25 mg of chloramphenicol and 0.03 grams of pyridoxal-5'-phosphate (a co-enzyme for SGOT, used for activating SGOT) in approximately 950 ml of deionized water. The pH of this solution was adjusted to 8.3 with approximately 1 ml of a solution of 5% TRIS (5grams of TRIS in 100 ml of deionized water), and the final volume was made to one liter with deionized water, which made a solution that was approximately 0.4 millimolar TRIS. This solution was filter sterilized, and was determined to be stable for at least one month when stored at 4°C.

c. Stabilized NADH III:

A stabilized NADH reagent was prepared by dissolving 0.44 grams of NADH and 25 mg of chloramphenicol in approximately 900 ml of deionized water. The pH of this solution was adjusted to 8.3 with 0.8 ml of a 0.1 molar solution of triethanolamine (TEA), and the final volume made to one liter with deionized water. (This calculates to be an 80 micromolar TEA solution.) This solution was filter sterilized, and was determined to be stable for at least one month when stored at 4°C.

d. Stabilized NADH reagent IV:

A stabilized NADH reagent was prepared by dissolving 0.188 grams of NADH and 25 milligrams of chloramphenicol in approximately 950 ml of deionized water. The pH of this solution was adjusted to 8.3 with approximately 0.4 ml of a solution of 5% imidazole (5 grams of imidazole in 100 ml of deionized water). The final volume of the reagent was made to one liter with deionized water. This calculates to be a solution of approximately 0.3 millimolar imidazole. The solution was filter sterilized, and was determined to be stable for at least one month when stored at 4°C.

e. Stabilized NADH V:

A stabilized NADH reagent was prepared by dissolving 0.298 g of NADH and 25 milligrams of chloramphenicol in approximately 950 ml of deionized water. The pH of this solution was adjusted to 9.2 with approximately 4 ml of 1 M TEA, and the reagent made to final volume of one liter with deionized water. This calculates to be approximately a 4 millimolar TEA solution. This solution was filter sterilized, and was determined to be stable for at least one month when stored at 4°C.

f. Stabilized NADH VI:

A stabilized NADH reagent was prepared by dissolving 0.328g of NADH and 25 mg of chloramphenicol in approximately 950 ml

of deionized water. The pH of this solution was adjusted to 8.3 with approximately 0.8 ml of 0.1 M TEA. The reagent was made to a final volume of one liter with deionized water and filter sterilized. This calculates to be approximately an 80 micromolar TEA solution. This solution is stable for at least one month when stored at 4°C.

g. Stabilized NADH VII:

A stabilized NADH reagent was prepared by dissolving 0.188 grams of NADH and 25 milligrams of chloramphenicol in approximately 950 ml of deionized water. The pH of this solution was adjusted to 8.3 with approximately ml of a 0.01 M solution of the strong base sodium hydroxide. This solution was made to a final volume of one liter with deionized water and filter sterilized. This calculates to be a 10 micromolar sodium hydroxide solution. This reagent was found to be stable for at least one month when stored at 4°C.

h. Stabilized NADH VIII:

A stabilized NADH reagent was prepared by dissolving 0.298 grams of NADH, 6 grams of TRIS and 25 milligrams of chloramphenicol in approximately 950 ml of deionized water. This solution was 50 millimolar with respect to TRIS. The pH of this solution was found to be 10. The volume was made to one liter with deionized water, and the solution was filter sterilized. This reagent is stable for one month when stored at 4°C.

i. Stabilized NADH IX:

A stabilized NADH reagent was prepared by dissolving 0.188 grams of NADH, 11 grams TEA and 25 mg chloramphenicol in approximately 950 ml of deionized water. This solution is 75 millimolar with respect to TEA. The pH of this solution was adjusted to 8.3 with approximately 15 ml of 1M hydrochloric acid. The final volume was made to one liter with deionized water, and the solution filter sterilized. This

reagent is stable for one month when stored at 4°C.

j. Stabilized NADH X:
Another stabilized NADH reagent was prepared as per embodiment IX, except that 0.44 g of NADH was used, that being the only change.

k. Stabilized NADH XI:
A stabilized NADH reagent was prepared by dissolving 0.188 grams of NADH and 25 mg of chloramphenicol in approximately 950 ml of deionized water. The pH of this solution was adjusted to 8.3 with approximately 1 ml of a 0.5% solution of diethanolamine (that is, 0.5 ml of diethanolamine (DEA) in 100 ml of deionized water). The final volume was made to one liter with deionized water. The concentration of DEA is approximately 50 micromolar. This reagent was filter sterilized, and was determined to be stable for at least one month when stored at 4°C.

l. Stabilized NADH XII:
A stabilized NADH reagent was prepared by dissolving 0.188 grams of NADH and 25 milligrams of chloramphenicol in approximately 950 ml of deionized water. The pH of this solution was adjusted to 8.3 with approximately 1 ml of a 0.01 M solution of the strong base ammonium hydroxide. This solution was made to a final volume of one liter with deionized water and filter sterilized. The concentration of ammonium hydroxide calculates to be approximately 10 micromolar. This reagent was found to be stable for at least one month when stored at 4°C.

m. Stabilized NADH XIII:
A stabilized NADH reagent was prepared by dissolving 0.188 grams of NADH and 25 milligrams of chloramphenicol in approximately 950 ml of deionized water. The pH of this solution was adjusted to 8.3 with approximately 1.6 ml of

a 0.01 M solution of rubidium carbonate, and the solution was made to a final volume of one liter with deionized water, and filter sterilized. The solution is approximately 16 micromolar rubidium carbonate. This reagent was found to be stable for at least one month when stored at 4°C.

VII. A. 2. NADPH Stabilization:

a. Stabilized NADPH I:

A stabilized NADPH solution was prepared by dissolving 0.334 g of beta nicotinamide-adenine dinucleotide phosphate, reduced form, tetra TRIS salt, trihydrate and 25 mg of chloramphenicol in approximately 950 ml of deionized water. The pH of this solution was adjusted to 9.2 with approximately 20 ml of 1.0 N sodium hydroxide. The final volume was made to one liter with deionized water. The concentration of sodium hydroxide calculates to be approximately 20 millimolar. This solution was filter sterilized, and was determined to be stable for at least one month when stored at 4°C.

b. Stabilized NADPH II:

A stabilized NADPH solution was prepared by dissolving 0.368 g of NADPH and 25 mg of chloramphenicol in approximately 950 ml of deionized water. The pH of this solution was adjusted to 8.3 with approximately 16 ml of 0.1 molar TEA, and the final volume was made to one liter with deionized water. This solution is approximately 1,6 millimolar TEA. This solution was filter sterilized, and was determined to be stable for at least one month when stored at 4°C.

VII. B. Stabilization of NADH and Enzymes, in Combination:

VII. B. 1. Embodiment of Stabilized NADH-LDH reagent:

A stabilized NADH reagent with lactate dehydrogenase (LDH, an enzyme which couples with the SGPT reaction to measure the pyruvate formed by the SGPT as observed by the loss of

absorbance of NADH) was prepared by dissolving 0.41 grams of NADH and 25 mg of chloramphenicol in approximately 900 ml of deionized water. The pH of this solution was adjusted to 8.3 with approximately 0.8 ml of 0.1M triethanolamine (TEA). After pH adjustment, 3600 units of lactate dehydrogenase were added. The reagent was brought to a final volume of one liter with deionized water and filter sterilized. This solution is stable for at least one month when stored at 4°C.

The NADH solution so prepared stabilizes the LDH enzyme, in this embodiment, whether the LDH is added prior to NADH stabilization or after the NADH stabilization.

VII. C. Embodiments of Stabilization of Enzymes in Working Reagents:

VII. C. 1. Stabilized LDH enzyme reagent:

A stabilized LDH enzyme reagent was prepared by dissolving 14.52 g of TRIS, 95 g of alanine, 0.2 g of disodium EDTA (ethylenediaminetetraacetic acid), 2o mg of chloramphenicol, and 1.69 g of alpha ketoglutaric acid, in approximately 800 ml of deionized water. The pH of this solution was adjusted to 7.4 with sulfuric acid, and 1200 units of LDH (lactate dehydrogenase) were added; and the final volume was made to one liter with deionized water. The solution was filter sterilized, and was determined to be stable for at least one month when stored at 4°C.

VII. C. 2. Stabilized MDH-LDH Working Reagents:

a. Stabilized MDH-LDH enzyme reagent I:

A stabilized enzyme reagent was prepared by dissolving 51.95 grams of potassium hydroxide, 99.9 grams of L-aspartic acid, 36.3 grams of TRIS, 8.1 grams of alpha ketoglutaric acid and 25 mg of chloramphenicol in approximately 750 ml of deionized water. The pH of this solution was adjusted to 7.4 with sulfuric acid. After pH adjustment,

14,000 units of malate dehydrogenase (MDH) and 2,080 units of lactate dehydrogenase (used for destroying pyruvate in the serum to avoid it being mistaken for SGOT activity) were added and the reagent made to volume of one liter with deionized water. This solution was filter sterilized, and was determined to be stable for at least one month when stored at 4°C.

In this embodiment, the potassium ions, the aspartate ions, the TRIS ions, and the sulfate ions, provide the stability of the enzymes in this working reagent. The L-aspartic acid and the alpha ketoglutaric acid are provided for the SGOT reaction, and the TRIS is a buffer for the reaction; and thus it is noted that the L-aspartic acid and the TRIS achieve a dual purpose, i.e. , that of enzyme stability as well as their role as a substrate in the SGOt reaction itself and as a buffer, respectively.

The inclusion of the potassium, aspartate, TRIS, and sulfate from the substances shown above is equivalent to the addition of a mixture of potassium and TRIS salts of aspartate and sulfate; and the solution contains about 1.2 equivalents of cation.

b. Stabilized MDH-LDH enzyme reagent II:

A stabilized enzyme reagent was prepared by dissolving 99.9 grams of L-aspartic acid, 8.1 grams of alpha ketoglutaric acid, 25 mg of chloramphenicol, 36.3 grams of TRIS, and 100 grams of beta glycerophosphate disodium salt in approximately 700 ml of deionized water. The pH of this solution was adjusted to 7.4 with potassium hydroxide. After pH adjustment, 14,000 units of malate dehydrogenase and 2,080 units of lactate dehydrogenase were added and the reagent brought to a total volume of one liter with deionized water. The solution was filter sterilized, and was determined to be stable for at least one month when

stored at 4°C.

In this embodiment, the potassium hydroxide and L-aspartic acid and the beta-glycerophosphate disodium salt provide stability for both the enzymes. The potassium hydroxide serves the dual purpose of adjusting the pH, and providing enzyme stability by its potassium ions; and the L-aspartic acid sdrves the dual purpose of enzyme stability as well as its role in the SGOT reaction itself.

The inclusion of the aspartate, TRIS, potassium, sodium and glycerophosphate in this way is equivalent to the addition of a mixture of sodium potassium and TRIS salts of aspartate and glycerophosphate; and the solution contains about 1.5 equivalents of cation.

c. <u>Stabilized MDH-LDH enzyme reagent III:</u>
A stabilized enzyme reagent was prepared by dissolving 51.95 grams of potassium hydroxide, 99.9 grams of L-aspartic acid, 36.3 grams of TRIS, 8.1 grams of alpha ketoglutaric acid, and 25 mg of chloramphenicol. The pH of this solution was adjusted to 7.4 with the monosodium salt of PIPES (Piperazine-N, N'-bix(2-ethanesulfonic acid). After pH adjustment, 14.000 units of MDH and 2.080 units of LDH were added, and the reagent made to volume of one liter with deionized water. This solution was filter sterilized, and was determined to be stable for at least one month when stored at 4°C.

In this embodiment, the sodium ions, the potassium ions, the aspartate ions of the aspartic acid, the TRIS ions, and the PIPES ions provide the stability of the enzyme in this working reagent. The aspartate and alpha ketoglutararic acid are provided for the SGOT reaction, and the TRIS is a buffer for the reaction; and thus it is noted that the

L-aspartic acid and the TRIS here achieve a dual purpose, i.e. that of enzyme stability as well as their role in the SGOT reaction itself and their buffering effect.

The inclusion of potassium, sodium, aspartate, TRIS , and PIPES in this way is equivalent to the addition of a mixture of potassium, sodium, and TRIS salts of aspartate and PIPES; and the solution contains about two equivalents of cation.

d. Stabilized MDH-LDH enzyme reagent IV:
This reagent was prepared as in stabilized MDH-LDH reagent Number II, but changing the 100 grams of beta glycerophosphate disodium salt to 100 grams of alpha glycerophosphate disodium salt.

It was observed that this reagent exhibited the same characteristics as the previous reagent Number II with respect to function as well as stability.

e. Stabilized MDH-LDH enzyme reagent V:
This reagent was prepared the same as in stabilized MDH-LDH reagent Number II except that the 100 grams of beta-glycerophosphate disodium salt was changed to 100 grams of beta-glycerophosphate dipotassium salt.

It was observed that this reagent exhibited the same characteristics as the previous reagent Number II with respect to function as well as stability.

f. Stabilized MDH-LDH enzyme reagent VI:
This reagent was prepared as in stabilized MDH-LDH reagent Number II, but changing the 100 grams of beta-glycerophosphate disodium salt to 100 grams of alpha-glycerophosphate dipotassium salt.

It was observed that this reagent exhibited the same characteristics as the previous reagent Number II with respect to function as well as stability.

g. Stabilized MDH-LDH enzyme reagent VII:
This reagent was prepared as in stabilized MDH-LDH enzyme reagent Number II but changing the 100 grams of beta glycerophosphate disodium salt to 80 grams of sodium ethylene glycol monophosphate.

It was observed that this reagent exhibited the same characteristics as the previous reagent Number II with respect to funtion as well as stability.

h. Stabilized MDH-LDH enzyme reagent VIII:
This reagent was prepared as in stabilized MDH-LDH enzyme reagent Number II, but changing the 100grams of beta-glycerophosphate disodium salt to 80 grams of potassium ethylene glycol monophosphate.

It was observed that this reagent exhibited the same characteristics as the previous reagent Number II with respect to function as well as stability.

VII. C. 3. Stabilized HK and G-6-PDH Working Reagents:
a. Stabilized HK and G-6-PDH enzyme reagent I:
A stabilized enzyme reagent containing hexokinase (HK) and glucose-6-phosphate dehydrogenase (G-6-PDH) was prepared by dissolving 14.9 grams of triethanolamine (TEA), 100 grams of beta glycerophosphate disodium salt, 50 mg of chloramphenicol, 5 grams of bovine serum albumin (BSA) (Fraction V), 2.42 grams of adenosine triphosphate (ATP), 1.99 grams of nicotinamide-adenine dinucleotide (NAD), and 0.99 grams of magnesium sulfate heptahydrate. The pH of this solution was adjusted to 7.5 with sulfuric acid. After pH adjustment, 2,000 units of hexokinase and 3,000 units of

glucose-6-phosphate dehydrogenase were added. The reagent was made to a final volume of one liter with deionized water and filter sterilized. This solution is stable for at least one month when stored at 4°C.

In this embodiment, the TEA serves the dual function of providing a buffer and stabilizing of the enzymes. Stability of the enzymes is also provided by the beta glycerophosphate disodium salt, and the BSA. The ATP, the NAD, and the magnesium sulfate heptahydrate are used in one of the reactions for the determination of glucose. The sulfuric acid serves the dual role of adjusting a pH and providing the sulfate ions which also serve as stabilizers of the enzymes.

The inclusion of the TEA sulfate is equivalent to the addition of a mixture of sodium and TEA salts of glycerophosphate and sulfate; and the solution contains one equivalent of cation.

b. <u>Stabilized HK and G-6-PDH enzyme reagent II:</u>
A stabilized enzyme reagent containing hexokinase (HK) and glucose-6-phosphate dehydrogenase (G-6-PDH) was prepared by dissolving 37 g of sodium hydroxide, 54 g of succinic acid, 14.9 grams of triethanolamine (TEA), 50 mg of chloramphenicol, 5 grams of bovine serum albumin (BSA), 2.42 grams of adenosine triphosphate (ATP), 1.99 grams of nicotinamide-adenine dinucleotide (NAD), and 0.99 grams of magnesium sulfate heptahydrate. The pH of this solution was adjusted to 7.5 with sulfuric acid. After pH adjustment, 2000 units of hexokinase and 3000 units of glucose-6-phosphate dehydrogenase were added, and the reagent was made to a final volume of one liter with deionized water and filter sterilized. This solution is stable for at least one month when stored at 4°C.

In this embodiment, the TEA serves a dual function of providing a buffer as well as stability for the enzymes. The sodium hydroxide and the sulfuric acid provide the sodium ions and the sulfate ions that stabilize the enzymes. Stability of the enzymes is also provided by the BSA. The ATP, the NAD, and the magnesium sulfate heptahydrate are provided for the reaction for the determination of glucose.

The inclusion of sodium, succinate, TEA, and sulfate is equivalent to the addition of a mixture of sodium and TEA salts of succinate and sulfate; and the solution contains about one equivalent of cation.

c. Stabilized HK and G-6-PDH enzyme reagent III:
This reagent was prepared as in stabilized HK and G-6-PDH enzyme reagent Number II, but changing the 54 grams of succinic acid to 60 grams of malic acid.

d. Stabilized HK and G-6-PDH enzyme reagent IV:
A stabilized enzyme reagent containing hexokinase (HK) and glucose-6-phosphate dehydrogenase was prepared by dissolving 100 ml of glycerol, 50 mg of chloramphenicol, 2 g of bovine serum albumin (BSA), 50.9 grams of PIPES,9.6 g of dextrose, 3 g of adenosine monophosphate (AMP), 33.9 grams of creatine phosphate, and 2.66 grams of adenosine diphosphate (ADP) in approximately 700 ml of deionized water. The pH of this solution was adjusted to 6.8 with dilute hydrochloric acid. After pH adjustment, 4500 units of hexokinase and 6300 units of glucose-6-phosphate dehydrogenase were added, and the final volume was made to one liter with deionized water. The solution was filter sterilized, and was determined to be stable for at least one month when stored at 4°C.

Here, the PIPES serves as a buffer and also as a stabilizer of the enzymes. Stability of the enzymes is also provided by a synergistic effect of the glycerol and the BSA with the

sodium PIPES salt.

The use of salts alone, if used in higher concentration, was found to stabilize the enzymes in the working reagents; however, higher concentrations of salts were found to unduly depress the rate of the CPK reaction for which this working reagent is used. Correspondingly, the use of high concentrations of glycerol was found to stabilize the enzymes in the working reagent; however, such working reagent solutions have high viscosity and other undesirable features of concentrated glycerol solutions. However, the combination of the proper low salt concentration and low glycerol concentration had the effect not achieved by either alone, that is, of stabilizing the working enzyme reagent without unduly depressing the rate of the CPK reaction or suffering other detriments of a high glycerol content; and the working reagents so prepared were otherwise useful in the CPK assay.

The dextros, the creatine phosphate, the ADP and the enzymes are used in the coupled reaction for the determination of creatine phosphokinase. The AMP serves to inhibit adenylate kinase activity.

The inclusion of the monosium salt of PIPES and creatine phosphate disodium salt in this reagent is equivalent to the addition of a mixture of sodium salts of PIPES and creatine phosphate; and the solution contains 0.35 equivalent of cation.

VII. C. 4. Stabilized GLDH Working Reagents:

a. Stabilized glutamate dehydrogenase (GLDH) enzyme reagent I:

A stabilized glutamate dehydrogenase working reagent was prepared by dissolving 24.2 grams of TRIS, 2.9 grams of alpha ketoglutaric acid, and 52 grams of potassium hydroxide in approximately 600 ml of deionized water. The pH of this

solution was adjusted to 8.0 with sulfuric acid and 1800 units of GLDH were added. The reagent was brought to final volume of one liter with deionized water. This solution was filter sterilized, and was determined to be stable for at least one month when stored at 4°C.

In this embodiment, the potassium, the TRIS and the sulfate provide the stability for the enzymes in the working reagent. The TRIS is also the buffer for the reaction. The alpha ketoglutaric acid is provided for the GLDH reaction.

The inclusion of potassium, TRIS, and sulfate in this way is equivalent to the addition of a mixture of potassium and TRIS salts of sulfate; and the solution contains one equivalent of cation.

b. <u>Stabilized Glutamate dehydrogenase (GLDH) enzyme reagent II:</u>

A stabilized glutamate dehydrogenase working reagent was prepared by dissolving 24.2 grams of TRIS, 2.9 grams of alpha ketoglutaric acid, 0.3 g dithiothreitol and 52 grams of potassium hydroxide in approximately 600 ml of deionized water. The pH of this solution was adjusted to 8.0 with sulfuric acid and 1800 units of GLDH were added. The reagent was brought to final volume of one liter with deionized water, and was filter sterilized. This reagent is stable for one month when stored at 4°C.

In this embodiment, the potassium, the TRIS and the sulfate provide the stability for the enzymes in the working reagent. The TRIS is also the buffer for the reaction. The alpha ketoglutaric acid is provided for the GLDH reaction.

The inclusion of potassium, TRIS, and sulfate in this way is equivalent to the addition of a mixture of potassium and TRIS salts of sulfate; and the solution contains one

equivalent of cation.

c. Stabilized Glutamate dehydrogenase (GLDH) enzyme reagent III:

A stabilized glutamate dehydrogenase working reagent was prepared by dissolving 24.2 grams of TRIS, 2.9 grams of alpha ketoglutaric acid, and 26 grams of potassium hydroxide in approximately 600 ml of deionized water. The pH of this solution was adjusted to 8.0 with sulfuric acid, and 1800 units of GLDH were added. The reagent was brought to final volume of one liter with deionized water, and was filter sterilized. This reagent is stable for one month when stored at 4°C.

In this embodiment, the potassium, the TRIS and the sulfate provide the stability for the enzymes in the working reagent. The TRIS is also the buffer for the reaction. The alpha ketoglutaric acid is provided for the GLDH reaction.

The inclusion of potassium, TRIS, and sulfate from the substances shown above is equivalent to the addition of a mixture of potassium and TRIS salts of sulfate; and the solution contains about 0.7 equivalent of cation.

VII. C. 5. Stabilized Urease - GLDH Working Reagents:

a. Stabilized Urease-Glutamate Dehydrogenase (GLDH) enzyme reagent:

A stabilized urease-glutamate dehydrogenase (GLDH) working reagent was prepared by dissolving 14.5 grams of TRIS, 4.2 grams of EDTA, 1.2 grams of ADP, 4.96 grams of alpha ketoglutaric acid, and 5 grams of BSA in approximately 500 ml of deionized water. Another solution was prepared by dissolving 52 grams of potassium hydroxide in approximately 300 ml of deionized water and adjusting the pH to 8.0 with sulfuric acid. This solution was added to the first solution and mixed. The pH of the resultant solution was

adjusted to 7.5 with sulfuric acid and 10.000 units of urease and 20,000 units of GLDH were added and the final volume made to one liter with deionized water. This reagent was filter sterilized, and was determined to be stable for at least one month when stored at 4°C.

In this embodiment, the potassium, TRIS, and sulfate ions provide the stability of the enzymes in the working reagent. The TRIS is also the buffer for the reaction. The ADP is an activator for the GLDH and the BSA is a stabilizer. The alpha ketoglutaric acid is provided for the GLDH reaction.

The inclusion of potassium, TRIS, and sulfate in this way is equivalent to the addition of a mixture of potassium and TRIS salts of sulfate; and the solution contains one equivalent of cation.

VII. C. 4. Stabilized coenzyme sulfhydryl reagent:
A stabilized coenzyme reagent was prepared by dissolving 1.49 grams of magnesium sulfate heptahydrate, 0.65 grams of dithiothreitol (DTT), 0.8 grams of ascorbic acid and 0.48 grams of NAD in approximately 900 ml of deionized water. The pH of this solution was adjusted to 5.0 with potassium hydroxide, and the final volume was made to one liter with deionized water. This solution was filter sterilized, and was determined to be stable for at least one month when stored at 4°C.

In this embodiment, the ascorbic acid provides stability for the dithiothreitol. The magnesium sulfate heptahydrate, the dithiothreitol (by activating the enzyme CPK) and the NAD all take part in the coupled reaction for the determination of creatine phosphokinase.

VII. D. Assay Procedures Using the Combination of Stabilization of NADH and/or NADPH and also Enzymes of Working Reagent Solutions:

VII. D. 1. Assay for SGOT by Stabilized Reagents:

a. Assay Procedures for SGOT Assay I:

In performing an SGOT assay, 0.5 ml of several of the stabilized MDH-LDH working reagents I-VIII were separately combined with 2.5 ml of several of NADH reagents I, IV, VII, IX, XI, XII, and XIII. The mixture in each case was warmed to 37°C. The 200 microliters of serum were added to this mixture and mixed well to assure homogeniety. The solution was transferred to a spectrophotometer, and the change in absorbance at 340 nm was recorded for three minutes. The amount of SGOT in a patient's serum was calculated by use of the following formula:

$$U/liter= \frac{\Delta A/min \times 1000 \times TV \times 1000 \times Tf}{6.22 \times 10^3 \times LP \times SV}$$

where:

$\Delta A/min$ = absorbance change per minute
1000: converts milliliters to liters
TV = total reaction volume or 3.2 ml
1000: converts millimoles to micromoles
Tf = temperature conversion factor (1.0 at 37°C)
$6.22 \times 10^3$ = molar extinction coefficient of NADH (340 nm)
LP = light path in centimeters
SV = sample volume (0.20 ml)
U = International Units of enzyme activity, defined as the amount of enzyme which converts one micromole of substrate in one minute at standard conditions.

Alternatively, the amount of SGOT in a patient's serum could be calculated from a known standard that was assayed in the manner just described for a patient. The relationship between the standard and the patient is expressed as follows:

$$\left(\text{U of SGOT in known standard}\right)\left(\frac{\Delta A \text{ patient}}{\Delta A \text{ standard}}\right) = \text{U of SGOT in patient's serum}$$

b. Assay Procedures for SGOT Assay II:
In another application using any of stabilized MDH-LDH reagent I - VIII and stabilized NADH reagent II, 200 microliters of a patient's serum was added to 2.5 ml of the stabilized NADH reagent, and allowed to incubate at 37°C for 5 minutes. Then 0.5 ml of prewarmed stabilized MDH-LDH enzyme reagent (prewarmed to 37°C) was added and the resultant mixture thoroughly mixed to insure homogeniety. The reaction mixture was then transferred to a spectrophotometer and the loss of absorbance at 340 nm (by the oxidation of NADH to NAD) was measured for a period for three minutes. The amount of SGOT in a patient's serum was calculated from the formula using the molar extinction coefficient as the previous example or from the calculation involving a known standard as in the previous example.

(This prodedure, using the stabilized NADH reagent referred to herein as NADH reagent II, containing pyridoxal-5'-phosphate, is different from the previous embodiment in that an incubation period is needed for the saturation of the SGOT with the pyridoxal-5'-phosphate not used in the NADH reagents I, IV, VII, IX, XI, XII, or XIII of the previous SGOT assay.)

VII. D. 2. Assay for SGPT by Stabilized Reagents:
a. Assay Procedure for SGPT Assay I:
For the SGPT assay, 1.0 ml of stabilized NADH-LDH reagent was combined with 2.0 ml of alanine reagent (described below) and warmed to 37°C. Then 200 microliters of serum was added to this combination, and mixed to assure homogeniety. The solution was transferred to a spectrophotometer, and

the absorbance change at 340 nm for 5 minutes was recorded. From the linear portion of the assay, the change in absorbance per minute was calculated. The amount of SGPT in the serum was calculated from the formula as in SGOT Assay I or from the calculation involving a known standard as in that SGOT Assay I.

The alanine reagent for this procedure was prepared by dissolving 14.52 grams of TRIS, 95 grams of L-alanine, 1.69 grams of alpha ketoglutaric acid, 0.2 grams of disodium ethylenediaminetetraacetic acid (EDTA), and 25 mg of chloramphenicol in approximately 700 ml of deionized water. The pH of this solution was adjusted to 7.4 with sulfuric acid. The final volume of the reagent was brougth to one liter with deionized water, and the solution was filter sterilized and stored in the refrigerator.

b. Assay Procedure for SGPT Assay II:

For the SGPT assay, 1.0 ml of stabilized NADH reagent III (the NADH reagent X was also used successfully) was combined with 2.0 ml of stabilized LDH enzyme reagent, and the mixture warmed to 37°C. Then 200 microliters of serum were added to this mixture, and the solution was mixed to assure homogeniety. The solution was then transferred to a spectrophotometer and the absorbance change at 340 nm was recorded. The amount of SGPT in the serum was calculated from the formula involving change in absorbance per minute, as in SGOT assay I, or from the calculation involving a known standard as in the SGOT assay I.

VII. D. 3. Assay Procedure for Glucose:

For assaying for glucose, 20 microliters of serum or standard was added to 3.0 ml of stabilized HK and G-6-PDH reagent I, II, or III, which had been prewarmed to 37°C, and mixed. The reagent and serum were incubated together for 5 minutes at 37°C. This mixture was then transferred

to a spectrophotometer that had had the optical density (O.D.) adjusted to read zero at 340 nm when 3.0 ml of stabilized hexokinase working reagent containing 20 microliters of 0.85% sodium chloride was read, and the O.D. of the solution containing serum was then read. The amount of glucose in the serum can be calculated from this absorbance and the absorbance of a standard that had been read according to the following equation:

$$\text{Glucose concentration of standard} \times \left(\frac{\text{O.D. serum}}{\text{O.D. standard}}\right) = \text{glucose concentration of serum}$$

VII. D. 4. Assay Procedure for CPK Enzyme Reagent:
In this assay, 1.0 ml of stabilized HK and G-6-PDH IV enzyme reagent was combined with 2.0 ml of stabilized coenzyme sulfhydryl reagent and warmed to 37°C. Then 100 microliters of serum or standard was added to the prewarmed mixture, and allowed to incubate for two minutes, then transferred to a spectrophotometer; and the change in absorbance at 340 nm was recorded for 3 to 5 minutes above a reagent blank. The change in absorbance per minute was calculated, and the units of CPK activity were calculated by using the standard's change in absorbance, in the following equation:

$$\text{units CPK in standard,} \times \left(\frac{\Delta \text{ A serum}}{\Delta \text{ A std.}}\right) = \text{units CPK in patient's serum}$$

VII. D. 5. Assay Procedure for LDH:
For this assay, 1.0 ml of stabilized NADH reagent III was added to 2.0 ml of lactate dehydrogenase (LDH) substrate reagent (described below), and the mixture prewarmed to 37°C. (Stabilized NADH reagent X was also used with the LDH substrate reagent to provide the reagent mixture, also warmed to 37°C.) Then 100 microliters of serum was added

to the warm reagent mixture and mixed thoroughly, then transferred to a spectrophotometer. The change in absorbance for three minutes at 340 nm was recorded, and the change in absorbance per minute was calculated. The units of LDH in the serum were calculated from the following equation:

$$U/liter = \frac{\Delta A/min \times 1000 \times TV \times 1000 \times Tf}{6.22 \times 10^{3} \times LP \times SV}$$

where:

$\Delta$ A/min = absorbance change per minute
1000: converts milliliters to liters
TV = total reaction volume or 3.1 ml
1000: converts millimoles to micromoles
Tf = temperature conversion factor (1.0 at 37°C)
$6.22 \times 10^{3}$ = molar extinction coefficient of NADH at 340 nm
LP = light path in centimeters
SV = sample volume (0.1 ml)

Alternatively, a standard can be recorded and the amount of LDH in the serum calculated from the change in absorbance of the serum compared to the change in absorbance of the standard according to the second formula shown for the SGOT Assay I.

The LDH substrate reagent was prepared by dissolving 17.3 grams of (dibasic potassium phosphate, 0.11 grams of pyruvic acid, and 25 mg chloramphenicol, in approximately 800 ml of deionized water. The pH was adjusted to 7.5 with hydrochloric acid, and the solution was filter sterilized and stored in the refrigerator.

VII. D. 6. Assay Procedure for GLDH Assay:
For the GLDH assay, 1.5 ml of the stabilized NADH reagent V or VIII (NADPH reagent I was also used successfully) was

combined with 1.5 ml of Substrate reagent (described below) and warmed to 37°C. After warming, 200 microliters of serum were added to this combination, and mixed to assure homogeniety. The solution was transferred to a spectrophotometer, and the absorbance change at 340 nm was recorded. The amount of GLDH in a patient's serum was calculated by use of the following formula:

$$U/liter = \frac{\Delta A/min \times 1000 \times TV \times 1000 \times Tf}{6.22 \times 10^3 \times LP \times SV}$$

where:

U = international unit of enzyme activity
$\Delta$ A/min = absorbance change per minute
1000: converts milliliters to liters
TV= total reaction volume or 3.2 ml
1000: converts millimoles to micromoles
Tf: temperature conversing factor (1.0 at 37°C.)
$6.22 \times 10^3$ = molar extinction coefficient of NADH at 340 nm
LP = light path in centimeters
SV = sample volume (0.2 ml)

The Substrate reagent for this precedure was prepared by dissolving 14.9 grams of TEA, 2.04 grams of alpha ketoglutaric acid, 15.42 grams of ammonium acetate, 1.69 grams of EDTA and 0.946 grams of ADP in approximately 750 ml of deionized water. The pH of this solution was adjusted to 8.0 with acetic acid, and the final volume made to one liter with deionized water. This solution was filter sterilized and stored in the refrigerator.

VII. D. 7. <u>Assay Procedure for Serum Ammonia:</u>
In performing the serum ammonia assay, samples must first be deproteinated by the following procedure:

Cool samples in an ice bath, and deproteinize with an equal volume of trichloroacetic acid solution (10 g trichloroacetic acid in 100 ml of deionized water). After 5 to 10 minutes centrifuge off the precipitated protein and neutralize the supernatant fluid with potassium hydrogen carbonate solution (20 g $KHCO_3$ in 100 ml of deionized water).

To perform the ammonia assay, 1.5 ml of the stabilized NADH reagent V (NADPH reagent I was also used successfully) was combined with 1.5 ml of stabilized glutamate dehydrogenase enzyme reagent I, (II and III were also found to function), and the mixture warmed to 37°C. After warming, 200 microliters of deproteinated serum was added. The mixture was transferred to a spectrophotometer, and the change in absorbance per minute at 340 nm was recorded. The amount of GLDH in a patient's serum was calculated from the equation as in the assay procedure for SGOT.

VII. D. 8. Assay Procedure for Blood Urea Nitrogen (BUN): To perform the BUN assay, 1.5 ml of the stabilized NADH reagent VI (stabilized NADPH reagent II was also used successfully) was combined with 1.5 ml of the stabilized urease-glutamate dehydrogenase working reagent, and warmed to 37°C. After warming, 20 microliters of serum were added to the mixture, and the mixture was transferred to a spectrophotometer where the change in absorbance per minute at 340 nm was recorded. A BUN standard was also run in the procedure just described, and the concentration of BUN in the patient's serum was calculated from this standard according to the following equation:

$$\text{Urea Nitrogen} = \Delta A/\text{min} \times S \times D$$

where:

$\Delta A$ = change in absorbance at 340 nm.

S = calculation factor derived from the standard (see below)

D = dilution factor

$$S = \frac{\text{concentration of the standard}}{\Delta A/\text{min of the standard}}$$

VIII. CONCLUSION:

Accordingly, it will thus be seen from the foregoing description of the inventiòn according to the embodiments of the invention herein set forth, that the present invention provides a new and useful means for the highly advantageous stabilization of working solutions of NADH, NADPH, and enzymes, in their working reagent use in several assays and procedures illustrative of and embodying the inventive concepts.

The objects and advantages of the invention are thus accomplished and achieved; and the details of the concepts are illustrated in the various embodiments.

It will be unterstood that certain modifications and variations of the specific and general concepts of the invention may be effected without departing from the concepts heretofore described; accordingly, the invention is not be considered limited to the specific form or embodiments set forth herein for the purpose of disclosing and illustrating the inventive concepts discovered and herein applied.

For example, there are a wide variety of antimicrobials which may be advantageously used in practicing the present invention, and are of course desirable the present invention, and are of course desirable to keep microbial growth out of reagents; provided, however, that they are such that the action of the enzymes is not inhibited, nor do the antimicrobials have unussually high absorbance levels at the wave length at which spectrophotometric measurements are to be made, nor do they react with an ingredient of the assay.

Further, it is of course desirable to use raw material enzymes for inclusion in the stabilized enzyme working solutions, which are desirably free or relatively free from proteolytic enzymes; for it is well known that proteolytic enzymes tend to destroy working reagent enzymes when in solution therewith.

LIST OF ABBREVIATIONS

| | |
|---|---|
| ADP | Adenosine diphosphate |
| AMP | Adenosine monophosphate |
| ATP | Adenosine triphosphate |
| BSA | Bovine serum albumin |
| BUN | Blood urea nitrogen |
| CPK | Creatine phosphokinase |
| DEA | Diethanolamine |
| DTT | Dithiothreitol |
| EDTA | Ethylenediaminetetraacetic acid |
| G-6-PDH | Glucose 6-phosphate dehydrogenase |
| GLDH | Glutamate dehydrogenase |
| HK | Hexokinase |
| INT | 2-(p-Iodophenyl)-3-(p-nitrophenyl)-5-phenyl Tetrazolium |
| LDH | Lactate dehydrogenase |
| MDH | Malic dehydrogenase |
| NAD | Nicotinamide adenine dinucleotide |
| NADH | Nicotinamide adenine dinucleotide (reduced form) |
| NADP | Nicotinamide adenine dinucleotide phosphate |
| NADPH | Nicotinamide adenine dinucleotide phosphate /reduced form) |
| PIPES | Piperazine-N,N'-bix(2-ethanesulfonic acid) |
| SGOT | Serum glutamic-oxaloacetic transaminase |
| SGPT | Serum glutamic pyruvic transaminase |
| TEA | triethanolamine, an amine base |
| TRIS | An amine base used to prepare buffers |

# Claims

1. A means for prolonging the stable life of a working reagent solution of NADH or NADPH, comprising the inclusion of one or more selected from the group consisting of an amine base, a strong base, a base whose cation is considered to have a valence of positive one, a base whose cation is considered to have a valence of positive two, and a basic solution of a salt formed from a reaction of a strong base and a weak acid.

2. NADH/NADPH stabilization as set forth in Claim 1, in which the inclusion is of a strong base such that the pH of the working reagent solution is raised to the basic range of at least about 7.9.

3. NADH/NADPH stabilization as set forth in Claim 2 in which the strong base is one or more selected from the group consisting of NaOH, LiOH, KOH, and $NH_4OH$.

4. NADH/NADPH stabilization as set forth in Claim 2, in which the strong base is RbOH.

5. NADH/NADPH stabilization as set forth in Claim 2, in which the strong base is one or more selected from the hydroxides of Group 1a elements of the Periodic Table, and from ammonium hydroxide.

6. NADH/NADPH stabilization as set forth in Claim 2, in which the strong base is one or more selected from the hydroxides of Group 1b elements of the Periodic Table.

7. NADH/NADPH stabilization as set forth in Claim 2, in which the strong base is one or more selected from the hydroxides of Group 1a elements of the Periodic Table, from ammonium hydroxide, and from the hydroxides of Group 1b elements of the Periodic Table.

8. NADH/NADPH stabilization as set forth in Claim 1, in which the inclusion is of basic solutions of salts which are formed from a reaction of a strong base and a weak acid, such that the pH of the working reagent solution is raised to the basic range of at least about 7.9.

9. NADH/NADPH stabilization as set forth in Claim 8, in which the salts are selected as one or more selected from the group consisting of rubidium carbonate, sodium borate, and sodium carbonate.

10. NADH/NADPH stabilization as set forth in Claim 1, in which the inclusion is of an amine base in such an amount that its concentration in the NADH or NADPH solution is less than 100 millimolar, and wherein the pH of the solution is at least about 7.9.

11. NADH/NADPH stabilization as set forth in Claim 1, in which the inclusion is of an amine base in such an amount that its concentration in the NADH or NADPH solution is less than 50 millimolar, and wherein the pH of the solution is at least about 7.9.

12. NADH/NADPH stabilization as set forth in Claim 1, in which the inclusion is of an amine base in such an amount that its concentration in the NADH or NADPH solution is less than 25 millimolar, and wherein the pH of the solution is at least about 7.9.

13. NADH/NADPH stabilization as set forth in Claim 1, in which the inclusion is of an amine base in such an amount that its concentration in the NADH or NADPH solution is less than 10 millimolar, and wherein the pH of the solution is at least about 7.9.

14. NADH/NADPH stabilization as set forth in Claim 1, comprising the inclusion of an amine base in such an amount that its concentration in the NADH or NADPH solution is less than 1 millimolar, and wherein the pH of the solution is at least about 7.9.

15.NADH/NADPH stabilization as set forth in Claim 12, 13, or 14, in which the amount of the amine base added is such that the pH is raised to at least about 7.9, and in which there is no conjugate acid of the amine base formed from the amine base other than that which may be formed by the NADH or NADPH.

16. NADH/NADPH stabilization as set forth in any of Claims 10 through 15, in which the amine base is selected from the group consisting of imidazole, TRIS, TEA, and DEA.

17. A means for stabilizing all the constituents NADH and/or NADPH, in an assay for SGOT, and also stabilizing whatever pyridoxal-5'-phosphate and LDH is/are present in the working solution of NADH and/or NADPH, in such assay, and also stabilizing the enzyme(s) MDH and/or LDH in working reagent solutions in such assay, comprising the combination of the inclusion of only a small amount of a base to the working reagent solution of NADH and/or NADPH and whatever pyridoxal-5'-phosphate and LDH is/are present therein, with the inclusion of a salt in the working reagent solution containing the enzyme (s) MDH and/or LDH.

18. A means for stabilizing all the constituents NADH and/or NADPH, in an assay for SGOT, and also stabilizing whatever pyridoxal-5'-phosphate and LDH is/are present in the working reagent solution of NADH and/or NADPH, in such assay, and also stabilizing the enzyme(s) MDH and/or LDH in working reagent solutions, in such assay, comprising the combination of the inclusion of a salt in the working reagent solution containing the enzyme(s) MDH and/or LDH, with NADH stabilization by the means as set forth in any of Claims 1through 16.

19. A means for stabilizing the constituent NADH in working reagent solution, and also stabilizing the LDH in working reagent solution, in an assay for SGPT, comprising stabilization of the NADH according to any of Claims 1 through 16, the LDH being added to the NADH solution either prior to or subsequent to the said stabilization of the NADH.

20. The stabilization of a working solution of NADH, in an assay for SGPT, by the means set forth in any of Claims 1 through 16.

21. A means for stabilizing the NADH working solution used in an LDH assay, by the stabilization means as set forth in any of Claims 1 through 16.

22. A means for stabilizing the NADH or NADPH in a working solution, in an enzymatic assay to measure enzymes or substrates which are detected by oxidation of NADH or NADPH, and also stabilizing the enzymes employed as reagents in a working solution, in such assay, comprising the combination of the inclusion of only a small amount of a base to the working reagent solution of NADH or NADPH, with the inclusion of a salt in the working reagent solution containing the enzymes.

23. NADH/NADPH stabilization as set forth in Claim 22, in which the NADH or NADPH is stabilized by the means as set forth in any of Claims 1 through 16.

24. NADH/NADPH stabilization as set forth in either of Claims 22 or 23, in which the concentration of the cation or anion of the included salt in the said working reagent solution is equal to or greater than 1.0 but less than or equal to 2.0 equivalents per liter.

25. NADH/NADPH stabilization as set forth in either of Claims 22 or 23, in which the concentration of the cation or anion of the included salt in the said working reagent solution is

greater than 2.0 but less than 2.8 equivalents per liter.

26. NADH/NADPH stabilization as set forth in either of Claims 22 or 23, in which the concentraion of the cation or anion of the included salt in the said working reagent solution is greater than 0.2 but less than 1.0 equivalents per liter.

27. NADH/NADPH stabilization as set forth in any of Claims 22 through 26, the cation of which salt is selected as one or more from a group consisting of cations of sodium, potassium, rubidium, ammonia, lithium, and basic amines.

28. 28. NADH/NADPH stabilization as set forth in any of Claims 22 through 26, the anion of which salt is selected as one or more from a group consisting of anions of glycerol monophosphate, ethylene glycol monophosphate, sulfate, sulfonated organic acids including PIPES, carbonate, phosphate, borate, and dicarboxylic acids, monocarboxylic acids, dicarboxylic amino acids including alanine and aspartic acid, alpha-glycerophosphate, and beta-glycerophosphate.

29. The step of including a stabilized working reagent solution of NADH or NADPH, in an assay for ammonium ion, the NADH or NADPH having been stabilized by the means set forth in any of Claims 1, 2, 3 (except the use of $NH_4$ ion), and 4 through 16 with a stabilized working reagent solution of GLDH enzyme.

30. The stabilization of the NADH or NADPH by the means as set forth in any of Claims 1, 2, 3 (except not the $NH_4$ ion), and 4 through 16 in working reagent solutions, in an assay for urea, including a means for stabilizing both the GLDH and urease enzyme constituents of the urea assay, in working reagent solutions, comprising the inclusion of a salt in the solution whereby the enzymes are stabilized.

31. NADH/NADPH stabilization as set forth in Claim 30 in which the cation of said salt is selected as one or more from a group consisting of cations of sodium, potassium, rubidium, lithium and basic amines.

32. NADH/NADPH stabilization as set forth in either of Claims 30 or 31 in which the anion of said salt is selected as one or more from a group consisting of glycerol monophosphate, ethylene glycol monophosphate, sulfate, sulfonated organic acids including PIPES, carbonate, phosphate, borate, and dicarboxylic acids, monocarboxylic acids, dicarboxylic amino acids including alanine and aspartic acid, alpha-glycerophosphate, and beta-glycerophosphate.

33. NADH/NADPH stabilization as set forth in any of Claims 30, 31 or 32, in which the base used for stabilizing the NADH or NADPH is selected from the group consisting of imidazole, TRIS, TEA, and DEA.

34. NADH/NADPH stabilization as set forth in either of Claims 17 or 18 in which the concentration of cation or anion of the included salt in the said working reagent solution containing the enzyme(s) is greater than 2.3 but less than 3.0 equivalents per liter.

35. NADH/NADPH stabilization as set forth in either of Claims 17 or 18, in which the concentration of cation or anion of the included salt in the said working reagent solution containing the enzyme(s) is greater than 0.5 but less than 1.0 equivalents per liter.

36. NADH/NADPH stabilization as set forth in any of Claims 17 or 18, in which the concentration of cation or anion of the included salt in the said working reagent solution containing the enzyme(s) is equal to or greater than 1.0 but equal to or less than 2.3 equivalents per liter.

37. NADH/NADPH stabilization as set forth in any of Claims 17, 18, 34, 35, or 36, in which the cation of which salt is selected as one or more from a group consisting of cations of sodium potassium, rubidium, ammonia, lithium, and basic amines.

38. NADH/NADPH stabilization as set forth in any of Claims 17, 18, 34, 35, or 36, in which the anion of which salt is selected as one or more from a group consisting of anions of glycerol monophosphate, ethylene glycol monophosphate, sulfate, sulfonated organic acids including PIPES, carbonate, phosphate, borate, and dicarboxylic acids except mallic acid, monocarboxylic acids, dicarboxylic amino acids including alanine and aspartic acid, alpha-glycerophosphate, and beta-glycerophosphate.

39. A process for prolonging the stable life of enzymes in a working reagent solution, comprising the inclusion, in said solution, of a salt which contains one or more of the following ions: the cations of sodium, potassium, rubidium, ammonia, lithium, and basic amines, and/or the anions of glycerol monophosphate, ethylene glycol monophosphate, sulfate, sulfonated organic acids including PIPES, carbonate, phosphate, borate, and dicarboxylic acids, monocarboxylic acids, dicarboxylic amino acids including alanine and aspartic acid, alpha-glycerophosphate, and beta-glycerophosphate.

40. Enzyme stabilization as set forth in Claim 39, in which the cation or anion concentration of the included salt in the said solution is greater than 1.5 but less than 2.5 equivalents per liter of working reagent solution.

41. Enzyme stabilization as set forth in Claim 39, in which the cation or anion concentration of the included salt in the said solution is equal to or greater than 0.5 equivalents per liter but less than or equal to 1.5 equivalents per liter of working reagent solution.

42. Enzyme stabilization as set forth in Claim 39, in which the cation or anion concentration of the included salt in the said solution is more than 0.2 equivalents per liter but less than 0.5 equivalents per liter of working reagent solution.

43. The stabilization of a working solution of the enzyme LDH, by the means set forth in any of Claims 39 through 42, in an assay for SGPT.

44. Enzyme stabilization as set forth in Claim 43, in which a working reagent solution of NADH is also stabilized by the inclusion of one or more selected from the group consisting of an amine base, a strong base, a base whose cation is considered to have a valence of positive one, a base whose cation is considered to have a valence of positive two, and a basic solution of a salt formed from a reaction of a strong base and a weak acid.

45. Enzyme stabilization according to any of Claims 39 or 42, in the process of the stabilization of the enzyme HK and the enzyme G-6-PDH in an assay for CPK, including the stabilization of the working reagent solution of DTT in said assay by inclusion of magnesium ions and/or ascorbic acid.

46. Enzyme stabilization as set forth in any of Claims 39 through 42, in the process of stabilizing both the HK and the G-6-PDH enzyme constituents of a glucose assay.

47. Enzyme stabilization as set forth in Claim 39, in a glucose assay, in which the concentration of the cation or anion of the included salt in the said working reagent solution is more than 2.0 but less than 2.8 equivalents per liter.

48. Enzyme stabilization as set forth in Claim 39, in which the concentration of the cation or anion of the included salt in the said working reagent solution is greater than or equal to 0.6 but equal to or less than 2.0 equivalents per liter.

49. Enzyme stabilization as set forth in Claim 39, in which the concentration of the cation or anion of the included salt in the said working reagent solution is greater than 0.2 but less than 0.6 equivalents per liter.

50. Enzyme stabilization as set forth in any of Claims 39 through 42, in a process of stabilization of the working reagent solution of GLDH in an assay for ammonium ion.

51. Enzyme stabilization as set forth in Claim 50, including the use of a stabilized working reagent solution of NADH or NADPH.

52. A process for prolonging the stable life of enzymes in a working reagent solution(s), in an assay for urea, in which both the GLDH and urease enzyme constituents of the urea assay are stabilized in working reagent solution(s) by the inclusion of a salt in their working reagent solution(3).

53. Enzyme stabilization as set forth in Claim 52 in which the cation of said salt is selected as one or more from a group consisting of cations of sodium, potassium, rubidium, lithium and basic amines.

54. Enzyme stabilization as set forth in either of Claims 52 or 53 in which the anion of said salt is selected as one or more from a group consisting of glycerol monophosphate, ethylene glycol monophosphate, sulfate, sulfonated organic acids including PIPES, carbonate, phosphate, borate, and dicarboxylic acids, monocarboxylic acids, dicarboxylic amino acids including alanine and aspartic acid, alpha-glycerophosphate, and beta-glycerophosphate.

55. Enzyme stabilization as set forth in any of Claims 52, 53, or 54, in which a base is used to stabilize the NADH or NADPH, said base being selected from the group consisting of imidazole, TRIS, TEA, and DEA.

56. Enzyme stabilization as set forth in any of Claims 39 or 42, in the stabilization of a working reagent solution of the enzyme HK and the enzyme G-6-PDH in an assay for CPK.

57. Enzyme stabilization as set forth in Claim 56 including the stabilization of the working reagent solution of DTT by inclusion of magnesium ions and ascorbic acid, such solution being of at least slightly acidic pH.

58. Enzyme stabilization as set forth in Claim 56 including the stabilization of the working reagent solution of DTT by inclusion of magnesium ions, such solution being of at least slightly acidic pH.

59. Enzyme stabilization as set forth in Claim 56 including the stabilization of the working reagent solution of DTT by inclusion of ascorbic acid, such solution being of at least slightly acidic pH.

60. The process of stabilizing a solution of DTT, by inclusion of ascorbic acid, such solution being of at least slightly acidic pH.

61. The process of stabilizing a solution of DTT, by inclusion of magnesium ions, such solution being of at least slightly acidic pH.

62. The process of stabilizing a solution of DTT, by inclusion of magnesium ions and ascorbic acid, such solution being of at least slightly acidic pH.

63. The invention as set forth in any of Claims 17, 18, 22, 23, 24, 25, 26, 27, 28, 30 through 59, in which the said salt or salts is/are used with the addition of known enzyme stability-enhancers including glycerol and/or BSA and/or reduced sulfhydryl compounds including DTT.